# EUROPÄISCHE PATENTSCHRIFT

(11) **EP 3 082 622 B1**
(45) Veröffentlichungstag und Bekanntmachung des Hinweises auf die Patenterteilung: **29.05.2019**
(21) Anmeldenummer: 14825283.6
(22) Anmeldetag: 16.12.2014
(51) Int. Cl.: A61B 17/16, B23C 1/20, B23C 5/24, B23Q 15/00, A61B 34/20

(54) **VERFAHREN UND SYSTEM ZUR STEUERBAREN VERSTELLUNG DER ABTRAGSLEISTUNG VON HANDGEFÜHRTEN MATERIAL- UND GEWEBETRENNENDEN WERKZEUGEN MIT EFFEKTOREN**
METHOD AND SYSTEM FOR THE CONTROLLABLE ADJUSTMENT OF THE MATERIAL REMOVAL RATE OF HAND-GUIDED MATERIAL-CUTTING AND TISSUE-CUTTING TOOLS COMPRISING EFFECTORS
PROCÉDÉ ET SYSTÈME DE RÉGLAGE COMMANDABLE DE LA PERFORMANCE D'ENLÈVEMENT D'OUTILS MANUELS À ENLÈVEMENT DE MATIÈRE ET DE TISSUS POURVUS D'EFFECTEURS

(30) Priorität: 17.12.2013 DE 102013226197; 14.04.2014 DE 102014105311
(43) Veröffentlichungstag der Anmeldung: 26.10.2016
(73) Patentinhaber: ALL-OF-INNOVATION GmbH, 85737 Ismaning (DE)
(72) Erfinder: MAIER, Thomas, 81679 München (DE); HEININGER, Sebastian, 85540 Haar (DE); LÖWE, Erik, 83404 Ainring (DE); LÜTH, Tim, 85737 Ismaning (DE)
(74) Vertreter: Gulde & Partner
(86) Internationale Anmeldenummer: PCT/EP2014/077945
(87) Internationale Veröffentlichungsnummer: WO 2015/091469

(56) Entgegenhaltungen:
- EP-A2- 1 346 694
- WO-A1-2010/046754
- DE-A1-102013 010 359
- DE-C- 364 851
- DE-C- 524 949
- FR-A- 919 350
- JP-A- S56 126 504
- US-A- 2 402 650
- US-A1- 2013 165 935

## Beschreibung

Die Erfindung betrifft ein Verfahren und ein System zur signal- oder positionsbasierten Steuerung der Abtragsleistung von handgeführten material- und gewebetrennenden Werkzeugen mit Effektoren.

Verfahren, System und Werkzeuge mit Effektoren sind insbesondere für die präzise Einhaltung von Arbeitsraumgrenzen beim Material- bzw. Gewebeabtrag mit Werkzeugen schnellrotierender Werkzeugmaschinen in der Chirurgie und im Manufakturbetrieb zur Herstellung von Freiformflächen geeignet.

Bei Manufakturarbeiten in Werkstätten und bei der interventionellen medizinischen Behandlung von Mensch und Tier werden handgeführte und handpositionierte Werkzeuge, häufig in der Form angetriebener Werkzeugmaschinen (z. B. Bohrmaschine mit Bohrer oder Fräse, elektrischer Hobelmaschine), zum Trennen und zum Abtrag von Material und Gewebe eines Objekts eingesetzt. Dies dient neben einer freien Formgebung meist auch der räumlichen Anpassung der Objektoberfläche an ein passgenaues Gegenstück mit definierten Begrenzungen. Gleichzeitig besteht die Notwendigkeit, bestimmte Strukturen oder Oberflächen des Objekts mit dem Werkzeug nicht zu verletzen, um so beispielsweise eine bereits abschließend bearbeitete Oberfläche oder Strukturen innerhalb des Objekts, die für das Auge des Anwender verborgen sind, zu schützen.

Aus dem Stand der Technik sind bisher als technische Hilfen für handgeführte und handpositionierte Werkzeuge die folgenden Verfahren bekannt:
a) Navigationsverfahren, die die Position und Orientierung von Werkzeugen relativ zum Objekt vermessen sowie grafisch und akustisch dem Anwender, Position, Orientierung und Abstand des handgeführten Werkzeugs zu den erlaubten Arbeitsraumgrenzen relativ zum Objekt anzeigen (Surgical Navigation, US 5389101, DE 19960020) sowie grafisch anzeigen, welche Gebiete am Objekt noch zu bearbeiten sind.
b) An dem Objekt befestigte Schablonen zur Begrenzung der freien Bewegung des handgeführten Werkzeugs innerhalb der erlaubten Arbeitsraumgrenzen relativ zum Objekt (Antike, Mittelalter, Surgical Template US 5141513).
c) Nachgiebige Mechanismen und Roboter, an denen das handgeführte Werkzeug befestigt ist. Diese Mechanismen und Roboter verändern relativ zu den erlaubten Arbeitsraumgrenzen die Freiheit, Reibung, Steifigkeit und Elastizität der handgeführten Bewegung am Objekt und applizieren sogar zusätzlich Kräfte und Momente zu denen der Handführung (Lueth, T. C. et al., "A surgical robot system for maxillofacial surgery," Industrial Electronics Society, 1998. IECON '98. Proceedings of the 24th Annual Conference of the IEEE, vol. 4, no., pp. 2470-2475).
d) Positions-, Orientierungs-, Abstands- und signalbasierte Abtragsleistungsbegrenzung relativ zum Objekt oder Strukturen des Objekts (Navigated Control, DE 000010117403C2).
e) Mechanismen zum linearen Heraus - und Hineinbewegen eines rotierenden Effektors relativ zu einer den Effektor umschließende Schutzhülse (Blue-Belt-Technologies, US 2012/0123418A1) zur Abtragsleistungsbegrenzung.

Verfahren zur Verstellung von Werkzeugschneiden sind aus der Literatur vorbekannt (DE 4401 496 A1) jedoch nicht für handgehaltene oder handgeführte Werkzeugmaschinen, deren Bewegungsabläufe der Werkzeugposition und -ausrichtung nicht über ein Programm bestimmt vorab festgelegt werden kann. Es ist kein Verfahren bekannt, das eine Werkzeugschneidenverstellung ohne Kopplung an eine programmierbare Bewegungssteuerung ermöglicht. Es ist kein Verfahren bekannt, das eine computergesteuerte oder positionsbasierte Werkzeugschneidenverstellung eines rotierenden Werkzeugs einer handgehaltenen oder handgeführten Werkzeugmaschine ermöglicht.

Aus (EP 0977644B1) ist ein Verfahren zum nachträglich Ausfahren der Schneiden bei Verschleiß der Schneiden bekannt.

Die EP 1346694 A2 beschreibt eine Fräsvorrichtung für Wirbelendflächen, wobei die Vorrichtung ein erstes Verstellelement mit einer ersten Gleitfläche und ein zweites Verstellelement mit einer zweiten Gleitfläche umfasst. Durch Bewegen der Verstellelemente zusammen oder auseinander, kann ein Effektor in eine Position höher oder niedriger auf der Gleitfläche bewegt werden.

Aus der US 2013/0165935 A1 ist eine orthopädische Reibahle bekannt mit einer ersten Welle und einer zweite Welle, die beweglich in wenigstens einem Abschnitt der ersten Welle angeordnet ist. Ein ein- und ausfahrbarer Effektor mit einer Vielzahl von steuerbaren Schneidelementen dient dem Materialabtrag.

Mit Bezug auf die Anwendung handgeführter angetriebener Werkzeuge mit Effektoren in der Chirurgie liegen durch den steigenden Bedarf des effizienten Einpassens von medizinischen Implantaten (z. B. Knie, Hüfte usw.) umfangreiche Erfahrungen der letzten 20 Jahre vor. Die Nachteile sind analog bei Manufakturarbeiten zur manuellen Bearbeitung von Material vorhanden. Die Nachteile des bekannten Stands der Technik sind die Folgenden:
a) Reine Navigationsverfahren mit grafischen, akustischen und taktilen, vibrierenden Rückmeldungen lösen nicht das Problem, dass der Anwender versehentlich bei Abrutschen oder mangelnder Konzentration das materialabtragende Werkzeug fehlerhaft ausrichtet und die gesetzten Arbeitsraumgrenzen verletzt. Je weniger Zeit für das Erlernen und die Durchführung der Anwendungen zur Verfügung steht, desto größer ist das Risiko. Das Risiko erhöht sich, wenn das Material unterschiedliche Dichten aufweist, das Werkzeug abgelenkt wird oder der Anwender in unergonomischer Haltung arbeitet sowie Stößen oder Kräften ausgesetzt ist.
b) Am Objekt befestigte Schablonen setzen voraus, dass eine ausreichend große Oberfläche zur Fixierung der Schablone vorhanden ist. Darüber hinaus entfalten Schablonen in erster Linie dann ihren Vorteil, wenn das Werkzeug in einer Vorzugsrichtung in die Schablone eingeführt wird. Es ist gegenwärtig nicht möglich eine dreidimensionale Freiformfläche zu fräsen und gleichzeitig eine dreidimensionale Beweglichkeit und eine dreidimensionale Ausrichtung der Werkzeuge zu ermöglichen. Dreidimensionale Freiformoberflächen sind darüber hinaus nur mit großem Aufwand herstellbar. Nicht eindeutig formschlüssig aufsetzbare Schablonen erfordern Navigationsverfahren zur Ausrichtung und Fixierung der Schablonen am Objekt. Schablonen benötigen immer zusätzlichen Platz am Objekt.
c) Nachgiebige (compliant-motion, hands-on) Roboter, an denen das handgeführte Instrument befestigt wird, haben einen begrenzten Arbeitsraum und müssen vor der Benutzung aufwändig relativ zum Objekt ausgerichtet werden. Im Betrieb erlauben hands-on Roboter die scheinbar freie Bewegung des Instruments. Die hohe Massenträgheit des Roboters gemessen am handgeführten Werkzeug oder Objekt sind selbst bei optimaler dynamischer Regelung eines hochdynamischen Antriebs ein begrenzender Faktor für die präzise Einhaltung der Arbeitsraumgrenzen. Sie sind darüber hinaus in der Beschaffung und im Betrieb teuer, benötigen einen eigenen Platz neben dem Objekt, und haben eine große Masse. Unter sterilen Arbeitsbedingungen müssen sie noch abgedeckt werden.
d) Das Verfahren der Positions-, Orientierungs-, Abstands- und signalbasierten Steuerung der Abtragsleistung (Navigated Control) ist zwar optimal geeignet die Arbeitsraumgrenzen einzuhalten, dennoch ist eine dynamisch steuerbare Veränderung der Abtragsleistung Voraussetzung, um zeitlich mit einer Schablone oder einer Mechanismen/Roboterlösung im Grenzbereich konkurrieren zu können. Ebenso kann die Eigenschaft der präzisen Einhaltung von Arbeitsraumgrenzen nur erreicht werden, wenn es einen möglichst steilen Übergang der Abtragsleistung des Werkzeugs gibt. Die Abschaltung der Leistung ist bei pneumatischen oder elektrischen Antrieben im aktuellen Stand der Technik bei hohem Massenträgheitsmoment des Werkzeugs problematisch, da im Schaltvorgang eine starke Kraftänderung auf die Hand ausgeübt wird.
e) Der Mechanismus zur Reduzierung der Abtragsleistung durch die Relativbewegung einer Schutzhülse um den Effektor erlaubt es die Abtragsleistung bei konstant laufender Drehzahl zu begrenzen. Da die Schutzhülse jedoch größer als der Effektor selbst ist und durch die Objektoberfläche blockiert werden kann, ist in der Praxis oft nur ein schnelles Einziehen oder Wegbewegen des Werkzeugs möglich. Hier werden ebenfalls Massen bewegt. Darüber hinaus muss die so erfolgte Relativbewegung mit der Hand früher oder später ausgeglichen werden. Dies behindert ein schnelles Arbeiten mit hoher Präzision.

Aufgabe der Erfindung ist es, die bekannten Nachteile des Standes der Technik zu vermeiden und ein Verfahrenund ein System für ein Werkzeug zu entwickeln, welche es erlauben, die Abtragsleistung von schnell rotierenden Werkzeugen zu steuern, ohne dass dafür die Drehzahl des Werkzeugs wesentlich verändert werden muss oder die Position, Orientierung oder die Geometrie des Werkzeugs mit Effektor wesentlich verändert wird.

Diese Aufgabe wird durch ein Verfahren gemäß den Merkmalen im Anspruch 1 und ein System gemäß den Merkmalen im Anspruch 8 gelöst. Zweckmäßige Ausgestaltungen der Erfindung sind in den Unteransprüchen enthalten.

Ein besonderer Vorteil der Erfindung besteht darin, dass auf die das Werkzeug mit dem Effektor führende Hand kein Ruck oder Schlag ausgeübt wird, indem die materialtrennenden Schneiden des Werkzeugs in ihrer Lage (Position und Orientierung) relativ zum Werkzeugeffektor mechanisch in mindestens zwei Lagen verstellt oder ein- und ausgefahren werden können bei gleichbleibender oder nur unwesentlich veränderter Drehzahl des Effektors. Dies erlaubt es, manuell schnell und mit hoher Präzision zu arbeiten. Ebenso ist es möglich, dass mindestens eine Schneidenabdeckung vor materialtrennenden Schneiden des Werkzeugs in ihrer Lage (Position und Orientierung) relativ zum Werkzeugeffektor mechanisch in mindestens zwei Lagen verstellt oder ein- und ausgefahren werden kann, um die Abtragsleistung der die materialtrennenden Schneiden des Werkzeugs zu verändern. Dabei ist es möglich, die Abtragsleistung des Effektors des Werkzeugs richtungsabhängig einzuschränken, um auf diese Weise eine Vorzugsrichtung oder eine Vorzugsvolumen beim Materialabtrag zu erzwingen, da das Werkzeug nur noch in der freigegebenen Abtragsvorzugsrichtung Material abtragen kann und weiter in das Objekt eindringen kann.

Das System ist einfach und preiswert herstellbar, zuverlässig einsetzbar und einfach zu warten, indem die am Werkzeug eingestellte Abtragsrichtung für den Benutzer am Werkzeug visuell (z.B. durch Einfärben der Schneidkanten) erkennbar ist oder akustisch oder grafisch (z.B. durch Anzeige an einem Bildschirm) signalisiert wird.

Die Veränderung der Abtragsleistung erfolgt in einem Zeitraum geringer als 1/10 Sekunde.

Weitere Vorteile der Erfindung resultieren daraus, dass die positionsbasierte Steuerung der Abtragsleistung auf einer kontinuierlich gemessenen Lage des handpositionierten oder handgeführten Werkzeugs und optional deren mathematischen Ableitungen sowie räumlichen Relationen zu definierten Grenzen relativ zu dem abzutragenden Material oder Gewebe oder einer Objektkörper-Messmarke beruht und dass die signalbasierte Steuerung der Abtragsleistung auf einem Sensorsignal oder Steuersignal beruht.

Ein weiterer Vorteil besteht darin, dass sich die Position und Orientierung des Effektors durch die Verstellung der Schneiden oder Schneidenabdeckung nicht oder nur in der Größenordnung der Schneidenlänge der Schneiden verändert und dass sich die räumliche Ausdehnung des Effektors durch die Verstellung der Schneiden oder der Schneidenabdeckung nicht oder nur in der Größenordnung der Schneidenlänge oder der Schneidenabdeckung verändert. Die Verstellung der Abtragsleistung erzwingt daher keine Positions- oder Lagekorrektur des Effektors. Die veränderte Abtragsleistung wird bei unveränderter Lage des Effektors erreicht.

Weiterhin ist es vorteilhaft, dass die erforderliche Energie zur Aufrechterhaltung der Signalverbindung und Steuerung des Stellantriebes direkt aus der Bewegungsenergie oder dem Antrieb der Werkzeugbewegung gewonnen wird. Ebenso ist es vorteilhaft, wenn die mit Abtragsgeschwindigkeit rotierenden Schneiden und/oder Schneidenabdeckungen von einem innenliegenden Stator durch mindestens eine verstellbare Betätigungslamelle nach außen gedrückt werden, um so nur einen Teil der Schneiden zum Abtrag freizugeben und auf diese Weise die Abtragsrichtung relativ zur Lage des Stators (oder der Werkzeugmessmarke) einzuschränken, wobei die Lage des Stators relativ zu der Werkzeugmessmarke bekannt ist und die Abtragsleistung des Effektors durch eine Verstellung richtungsgesteuert wird.

Verfahren und System sind insbesondere für die präzise Einhaltung von Arbeitsraumgrenzen beim Material- bzw. Gewebeabtrag mit Werkzeugen in schnellrotierenden Werkzeugmaschinen in der Chirurgie und im Manufakturbetrieb zur Herstellung von Freiformflächen vorteilhaft anwendbar. Die Veränderung der Abtragsleistung bei konstanter Drehzahl und Effektorlage machen eine sehr schnelle manuelle Werkzeugbewegung, beispielweise schraffierende Hin- und Herbewegungen, mit präzisen Abtragsgrenzen möglich sowie eine automatische Zentrierung und Richtungskorrektur des manuell geführten Effektors und damit des Werkzeugs.

Das Verfahren und ein entsprechendes System sowie ein Werkzeug sind ein weiterer elementarer Schritt die präzise Bearbeitungsleistung eines Menschen durch seine motorischen Fähigkeiten in Kombination mit einer signal-, positions-, orientierungs-, und abstandsbasierten schnellen Reduktion der Abtragsleistung eines von diesem handgeführten Werkzeugs zu erhöhen.

Durch die Verstellung der Schneiden ist es möglich, mit einem Werkzeug mehrere Werkzeuge mit geringerer Abtragsleistung zu emulieren und so die Zahl der Werkzeugwechsel zu reduzieren. Anstatt Effektoren mit geringeren Schneidenlängen zu verwenden, können zum Oberflächenfinish die wirksamen Schneidenlängen reduziert werden. Die Oberflächenqualität nimmt zu.

Das Anwendungsspektrum rotierender Werkzeuge wird auf Kosten anderer trennender Verfahren wie Laser wieder zunehmen, da sich die Abtragsleistung ähnlich schnell bzw. deutlich schneller als bisher verstellen lässt. Dadurch wird die Verwendung von manuell geführten Werkzeugen, die funktionsbedingt ein Massenträgheitsmoment besitzen oder durch die Wechselwirkung von Werkzeug und Material unerwünscht abgelenkt werden können, für Präzisionsaufgaben möglich.

Das Anwendungsspektrum handgehaltener und handgeführter Werkzeugmaschinen wird sich deutlich erhöhen, da der Mensch ähnliche Leistungen wie eine Werkzeugmaschine erbringen kann. Entsprechend können mehr Menschen eine höherwertige Tätigkeit ausüben, die bisher nur über Werkzeugmaschinen erbracht wird. Ebenso nimmt das Spektrum der manuellen Tätigkeiten zu bei denen eine präzise dreidimensionale Oberflächenbearbeitung erforderlich ist.

Bezogen auf die Anwendung in der Knochenchirurgie wird sich die Passgenauigkeit von orthopädischen Implantaten erheblich erhöhen. Es können kleiner Eingriffe mit höherer Präzision durchgeführt werden. Die Geschwindigkeit mit der orthopädische Implantate eingepasst (Präparation und Einpassung) werden kann sich erheblich erhöhen. Die reduzierte Zeit hat positiven Einfluss auf den Heilungsprozess der Patienten. Die wachsende Zahl v.a. älterer Menschen mit steigendem Bedarf für den Ersatz von Knie-, Hüft-, Schultergelenken wird mit einer konstanten oder sinkenden Zahl von Chirurgen versorgt werden können.

Die Kosten für die Anschaffung von Investitionsgütern (medizinische Roboter) zum Erreichen hoher Implantatleistungen werden erheblich sinken. Zum gegenwärtigen Kenntnisstand sind automatisierte Implantationsvorgänge wesentliche Voraussetzung dafür, um die durch die älter werdende Gesellschaft notendigen Gelenkersatzeingriffe bei konstanter oder abnehmenden Anzahl von Chirurgen sicherstellen zu können.

Es zeigen:
- Fig. 1: Handgeführtes Werkzeug mit einem rotierenden materialtrennenden Effektor,
- Fig. 2: Formtreue und positionsgenau in ein Objekt zu fräsende Kavität mit darunter verborgener empfindlicher Struktur,
- Fig. 3: Materialtrennender Effektor mit bei hoher Drehzahl ein- und ausfahrbaren Werkzeugschneiden,
- Fig. 4: Querschnitt durch einen materialtrennender Effektor, bei dem die Schneiden durch Gelenke verstellt werden können,
- Fig. 5: Beispielhafte Übertragung der Stellkräfte zur Schneidenverstellung während der Rotation des Werkzeugs mit Abtragsgeschwindigkeit,
- Fig. 6: Signal-, positions-, Orientierungs- und abstandsabhängige Steuerung der Werkzeugschneiden in Bezug zum bearbeiteten Material oder Gewebe, und
- Fig. 7: Querschnitt durch einen materialtrennenden Effektor, bei dem die Schneiden durch Schneidenabdeckungen in der Abtragsleistung reduziert werden können.

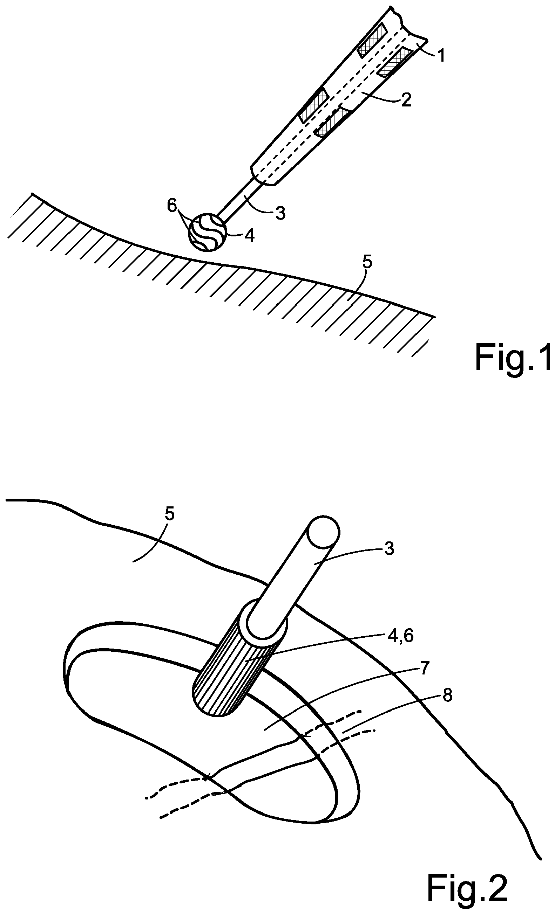

Fig. 1 zeigt ein handgeführtes angetriebenes Werkzeug 1 wie es zur Materialtrennung bzw. zur spanabhebenden Bearbeitung wie das Bohren, Fräsen, Sägen, Trennen usw. eingesetzt wird. Häufig werden pneumatische oder elektrische Antriebe verwendet, wenn hohe Drehzahlen für die notwendige Abtragsleistung erreicht werden müssen. Das Werkzeug 1 überträgt das Drehmoment für die Materialtrennung typischerweise über eine starre oder flexible Welle 3 mit der über ein Spannfutter 2 das materialtrennende Werkzeug 1 mit dem eigentlichen Werkzeugeffektor 4 verbunden ist. Der sich zur Schneidgeschwindigkeit angepasst drehende Effektor 4 wird auf das Material 5 oder Gewebe 5 aufgesetzt. Dabei trennen die Schneiden 6 am Effektor 4 das Material 5 auf und entfernen so einen Teil des Materials 5.

Fig. 2 zeigt eine Oberflächenstruktur in die eine passgenaue Kavität 7 eingefräst werden soll, ohne dabei versehentlich eine im Material 5 befindliche sensible Struktur 8 mit dem als Fräsereffektor ausgebildeten Effektor 4 zu verletzen. An der geplanten Begrenzung der Kavität 7 und in der Nähe der zu schützenden Struktur 8 kann die Abtragsleistung des Werkzeugs 1 und des Effektors 4 durch die Verstellung der Schneiden 6 reduziert werden, ohne dass dabei die Drehzahl des Werkzeugs 1 reduziert werden muss. Allenfalls erfolgt eine unwesentliche Reduzierung der Drehzahl. Auf diese Weise entsteht kein Ruck oder Schlag auf die das Werkzeug 1 führende Hand durch eine Drehzahlveränderung der drehenden Massen von Motor und Werkzeug 1.

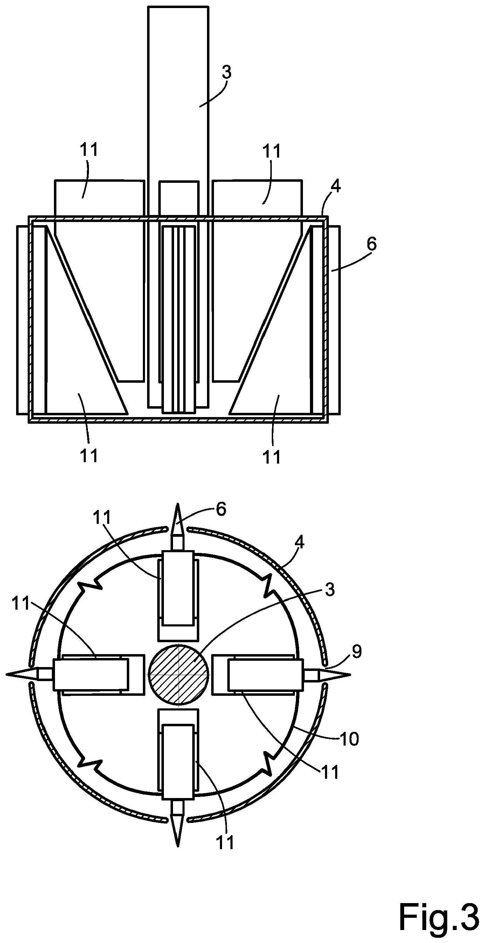

Fig. 3 zeigt in Seitenansicht und von unten eine beispielhafte Ausführung des materialtrennenden Werkzeugs 1 mit der Welle 3, dem Effektorkopf des Effektors 4 und den verstellbaren Schneiden 6. Die Figur zeigt vier Schneiden 6, die durch Öffnungen 9 aus dem Effektorkopf des Effektors 4 herausgedrückt bzw. hinein bewegt werden können. Die Öffnungen 9 zum Ausfahren der Schneiden 6 sind so ausgelegt, dass diese - bei eingefahrenen Schneiden 6 - auch bei hoher Drehzahl und direktem Kontakt des Effektors 4 mit der Oberfläche des abzutragenden Materials oder Gewebe 5 kein oder nicht nennenswert Material abtragen können. In Fig. 3 sind die Schneiden-Ausfahröffnungen 9 mit gerundeten Kanten dargestellt. Ein Einzugsmechanismus 10 stellt sicher, dass die Schneiden 6 selbst bei hoher Drehzahl und unter Kontakt nicht unbeabsichtigt eine materialtrennende Wirkung entfalten, sondern sich hinter die Öffnungen 9 Schutz zurückziehen. In Fig. 3 ist der Einzugsmechanismus 10 durch schneidenverbindende Federelemente ausgeführt.

Um die Schneiden 6 aus der Schutzposition zu drücken, wird ein Ausfahrmechanismus 11 benötigt. In Fig. 3 ist dieser als Keilmechanismus ausgelegt, der beim Niederdrücken eine radial wirkende Kraft auf die Schneiden 6 ausübt und diese nach außen in den Materialeingriff drückt. Alle in Fig. 3 dargestellten Elemente rotieren als Bestandteil des Effektorkopfes des Effektors 4.

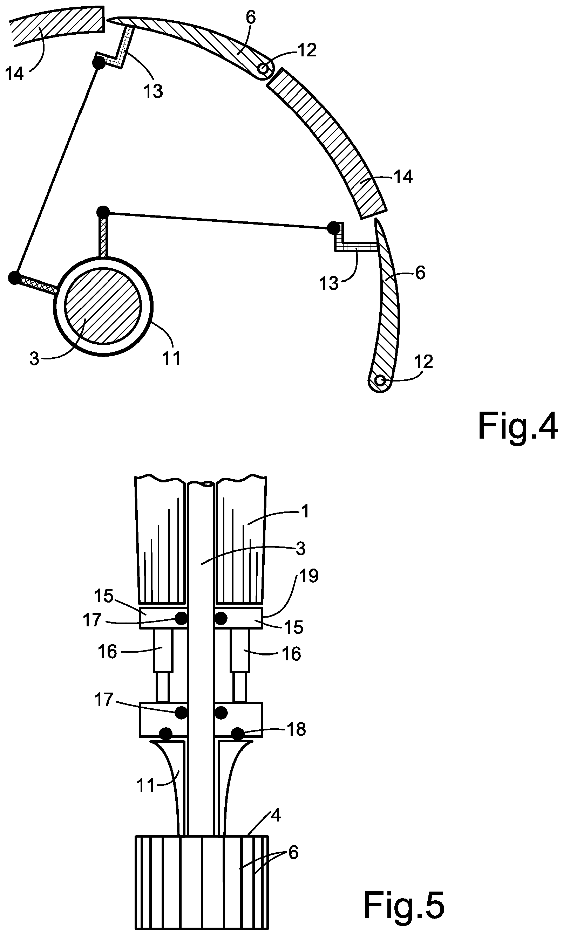

Fig. 4 zeigt eine weitere beispielhafte Ausführung des Werkzeugs 1, bei dem Schneiden 6 nicht eingezogen werden, sondern auf der Außenfläche des Effektors 4 mit Drehgelenken 12 angebracht sind. Sie können durch Druck von Innen nach Außen ausgestellt werden, wobei vorzugsweise ein Anschlag 13 das Ausbrechen der Schneiden 6 geeignet verhindert. Die Drehgelenke 12 sind vorzugsweise als Festkörpergelenke ausgeführt, die durch eine geeignete Schwächung bzw. Formgebung des Materials erzeugt werden können. Der Mechanismus der Schneiden 6 ist hier im Querschnitt dargestellt, jedoch können alle Elemente wie bei einem Bohrer in Form einer gestreckten Spirale (Helix) um einen zylindrischen Kern herumlaufen.

Der Ausfahrmechanismus 11 ist in Fig. 4 nicht als Keil ausgeführt, sondern als Gelenkkinematik, die durch eine Verdrehung des Ausfahrmechanismus 11 gegenüber der Welle 3 zu einem Ausstellen der Schneiden 6 über den Schneidenschutz 14 hinaus führt. Die Verdrehung des Ausfahrmechanismus 11 kann natürlich auch durch eine schneckenförmige Linearbewegung erreicht werden.

Fig. 5 zeigt eine beispielhafte Übertragung der Stellkräfte zur Verstellung der Schneiden 6 über einen Scheidensteueraufsatz 15. Der Schneidensteueraufsatz 15 ist dazu entweder direkt mit dem Handstück des Werkzeugs 1 verbunden oder wird gemeinsamen mit der Welle 3 und dem Schneidenausfahrmechanismus 11 beim Spannvorgang mit dem Spannfutter 2 verspannt. Um die Bewegung des Schneidensteueraufsatzes 15 von der Werkzeugbewegung zu entkoppeln, ist vorzugsweise ein Lager 17 angeordnet. Ebenso ist ein Lager 18 vorteilhaft, um die Schneidenstellbewegung von der Werkzeugbewegung und dem Schneidenausfahrmechanismus 11 zu entkoppeln. Die eigentliche Stellbewegung wird über einen Stellantrieb 16 erreicht, der hier in Fig. 5 als Linearmotor, beispielsweise als Piezomotor, ausgelegt ist, aber auch pneumatisch, hydraulisch oder elektrodynamisch ausgelegt werden kann.

Über ein weiteres Lager, das in Fig. 5 nicht zu sehen ist, könnte der Schneidenausfahrmechanismus 11 auch gezogen werden. Der Stellantrieb 16 würde dann auch ohne einen getrennt ausgeführten passiven Einzugsmechanismus 10 die Schneidenverstellung in beide Richtungen durchführen können.

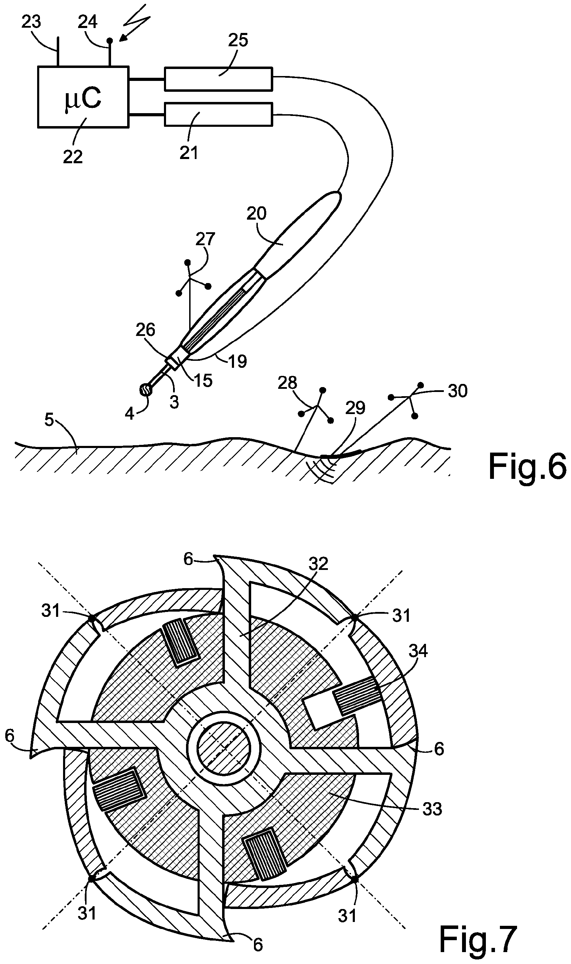

Fig. 6 zeigt den Maschinenantrieb 20, der über einen Leistungsverstärker bzw. eine Motorsteuerung 21 durch die Signale eines Steuerrechners 22 angesteuert werden kann. Die Drehzahlvorgaben des Steuerrechners 22 kommen über eine externe Signalleitung 23 oder eine Signalfunkstrecke 24, um beispielsweise die Drehzahl der Maschine über ein Fußpedal (nicht dargestellt) steuern zu können. Analog dazu dient eine Schneidenverstellsteuerung 25 des Stellantriebs zur Schneidenverstellung über die Steuersignalleitung 19. Auch hier ist es möglich, über die Signalleitung 23 oder die Signalfunkstrecke 24 die sofortige Verstellung der Schneiden 6 zu signalisieren, beispielsweise wenn der Effektor 4 die Grenzen des Arbeitsraumes verlässt. Zur Messung der Position und Orientierung des Werkzeugs 1 wird bekanntermaßen relativ zum Werkzeug 1 oder an der Maschine fixiert eine Messmarke (27) für ein Koordinatenmesssystem (nicht dargestellt) angebracht. Eine zweite Messmarke (28) befindet sich fixiert am abzutragenden Material oder Gewebe 5 des zu bearbeitenden Objektes. Die daraus resultierende Information über die relative Bewegung und die Grenzen zum Arbeitsraum werden entweder extern verarbeitet oder an den Steuerrechner 22 gesendet. Von dort aus wird entweder die Drehzahl oder die Schneidenstellung gesteuert. Die Messmarken 27, 28, 30 sind in Fig. 6 als Reflektoren für optische Koordinatenmesssysteme ausgelegt. Es könnte sich ebenso um Messmarken für ein elektromagnetisches Koordinatenmesssystem handeln. In diesem Fall ist vorzugsweise noch ein Drehzahlsensor 26 am Werkzeug 1 anzubringen, um die Frequenz der elektromagnetischen Störfelder am Entstehungsort zu empfangen und an den Steuerrechner 22 und das Koordinatenmesssystem weiterzuleiten, um diese dort über eine Bandsperre bei der Positionsmessung auszufiltern. Anstatt einer positionsbasierten Verstellung der Schneiden 6 relativ zu Arbeitsraumgrenzen ist auch die direkte Signal-, Bildverarbeitung eines signal- oder bildgebenden Systems zur Erzeugung der Schneidenverstellung möglich. Dazu wird ein signal- oder bildgebendes System 29, wie beispielsweise ein Nervmonitor oder ein mehrdimensionales bildgebendes Gerät, zur Signalerfassung eingesetzt. Hier kann dann im Signal oder Bild direkt erkannt werden, ob eine Schaltung der Schneiden 6 erforderlich ist.

Fig. 7 zeigt eine weitere Variante, bei der - vor dem Hintergrund der Krafteinleitung beim Materialabtrag - nicht die Schneide 6, sondern ein Schneidenschutz 14 mit einem Schneidenabdeckungsbereich 14a über ein als Drehgelenk oder ein Festkörpergelenk ausgebildetes Schneidenabdeckunsgelenk 31 gegenüber der Schneide 6 verstellt wird. Dabei wird bei Bedarf die Öffnung zwischen Schneidenschutz 14 und der Schneide 6 geschlossen. Es ist bei der Verwendung von weiteren - nicht dargestellten - Festkörpergelenken zwischen Schneidenabdeckung 14 und Schneide 6 auch möglich, die Oberfläche des Effektors 4 vollständig geschlossen zu gestalten, um das Eindringen von Partikeln in den Effektor 4 zu vermeiden. In der Figur 7 sind die Schneiden 6 fest und unverstellbar an einer Hohlwelle 32 befestigt, die um einen zentrierten Stator 33 mit Schneidgeschwindigkeit vorzugsweise an zwei Stellen ringförmig gelagert rotiert. Die Orientierung des Stators 33 relativ zum Werkzeug 1 bzw. zur Werkzeugmessmarke 27 ist bekannt. Der Schneidenschutz 14 ist mit seinem Schneidenabdeckungsgelenk 31 direkt an der Rückseite der Schneide 6 befestigt. Im ausgefahrenen Zustand verdeckt der Schneidenabdeckungsbereich 14a die Schneide 6 und drückt ebenfalls bei der Ausfahrbewegung Materialreste, die sich vor der Schneide 6 befinden, heraus. Ohne inneren Druck führt die radienfolgende Formgebung dazu, dass die Abdeckung in den Effektor 4 hineingedrückt wird und so die Schneiden 6 freigelegt werden. Im Stator 33 befindet sich in einer Ausnehmung mindestens eine verstellbare Betätigungslamelle 34, die aus der rotationssymmetrische Form des Stators 33 in mindestens eine Richtung aus- und eingefahren werden kann. Es sind weitere Varianten möglich, um den Stator 33 an mindestens einer Stelle in seinem Radius zu verändern. Durch das Ausfahren der Betätigungslamellen 34 am Stator 33 verliert das Werkzeug 1 in dieser Richtung seine Abtragswirkung. Werden Betätigungslamellen 34 in alle Richtungen verstellt, wird der gesamte Effektor 4 seine Abtragsleistung verlieren. Werden gezielt Betätigungslamellen 34 eingefahren, dann erhält der Effektor 4 relativ zum Werkzeug 1 bzw. zur Werkzeugmessmarke 27 eine ausrichtungsbegrenzte einstellbare Abtragsleistung. Durch geeignete Steuerung durch den Steuerrechner 22 kann damit die Abtragsleistung des Effektors 4 auf bestimmte Winkelsegmente begrenzt werden. Die Bewegung der Betätigungslamellen 34 kann nicht nur elektrisch sondern ebensogut hydraulisch oder pneumatisch erfolgen. Letzteres ist für pneumatische Antriebe besonders vorteilhaft.

Das Verfahren ermöglicht es, die Abtragsleistung von mindestens einer der materialabtragenden Schneiden 6 eines manuell geführten Werkzeugs 1 durch eine Verstellbewegung der Schneiden 6 oder eines Schneidenschutzes 14 zu reduzieren. Auf diese Weise kann bei gleichförmiger Werkzeugbewegung die Abtragsleistung des materialabtragenden Effektors 4 am Objekt verstellt bzw. reduziert werden. Durch die beibehaltene gleichförmige Werkzeugbewegung und konstante Drehzahl entstehen nur minimal Kräfte oder Momente auf die werkzeugführende Hand. Durch die kurzen Verstellwege können extrem kurzfristige Anpassungen der Abtragsleistungen ermöglich werden.

Unterschieden werden die signalbasierte Steuerung einerseits und die Steuerung der Abtragsleistung in Abhängigkeit von der Werkzeug-Pose (Position und Orientierung) andererseits.

Eine beispielhafte Anwendung der signalbasierte Steuerung ist die Folgende: Beim Bohren eines Loches mit einer elektrischen Bohrmaschine in eine Zimmerwand kann über einen signalgebenden Sensor erkannt werden, dass sich in Richtung des Bohrkanals eine elektrische Leitung oder einer Wasserleitung befindet, von denen bekannt ist, dass diese nicht durch den Bohrer verletzt werden dürfen. In diesem Fall wird sofort die Abtragsleistung des Bohrwerkzeugs reduziert, so dass die Leitungen nicht verletzt werden.

Eine weitere beispielhafte Anwendung ist die Folgende: Beim Bohren einer Kavität im Mastoidbereich des Schädelknochens wird über einen signalgebenden Sensor erkannt, dass sich in Richtung oder unmittelbarer Nähe des Bohrkanals eine Nervenbahn oder eine Blutgefäß durch den Knochen zieht, von denen bekannt ist, dass diese nicht durch den Bohrer verletzt werden dürfen. In diesem Fall wird sofort die Abtragsleistung des Bohrwerkzeugs reduziert, so dass die Leitungen nicht verletzt werden.

Beide Anwendungen sind als Sicherheitsmaßnahmen gedacht, die keine räumliche Vorplanung durch den Werker/Arzt erfordern sondern in denen ein Schaltsignal durch einen integrierten Sensor oder externen Sensor übermittelt wird. In diesen Fällen kann möglicherweise grundsätzlich ein manuelles Zuschalten der Abtragsleistung sinnvoll sein, um ein preiswertes Werkzeug 1 realisieren zu können, währen die Abschaltung signalbasiert erfolgt.

In anderen Fällen ist es vorteilhaft, analog dazu auch das Einschalten über ein Signal zu ermöglichen. In diesem Fall würde sich das Werkzeug 1 bereits mit der richtigen Drehzahl exakt an der richtigen Stelle befinden, wenn die Abtragsleistung zugeschaltet wird. Das bekannte Problem der Drehmomentübertragung durch Haftreibung und der daraus resultierenden Positionsänderung bei mangelnder manueller Haltekraft entfällt.

Eine beispielhafte Anwendung der werkzeugposenbasierte Steuerung analog zu der Leistungssteuerung aus (US 7346417) ist die Folgende: Um ein Implantatlager in Form einer Kavität in einen Oberschenkelknochen für ein künstliches Kniegelenk einzubringen ist es erforderlich, an einer bestimmten Position und Orientierung am Knochen eine definierte Freiformoberfläche in den Knochen einzufräsen. Sowohl die Zugangsöffnung als auch der Arbeitswinkel sind dabei stark eingeschränkt. Sobald sich der Effektor 4 des freihandgeführten Werkzeugs 1 an der Grenze des erlaubten Arbeitsraums befindet, wird die Abtragsleistung des Effektors 4 ohne eine dazu erforderliche Reduzierung der Werkzeugmaschinenleistung durch die Verstellung der Schneiden 6 bzw. der Schneidenabdeckung 14 reduziert. Durch das weitgehend konstante Massenträgheitsmoment durch die konstante Drehzahl der Werkzeugmaschine und des Effektors 4 des Werkzeugs 1 kann die Abtragsleistung an der Grenze des erlaubten Arbeitsraums sprunghaft geändert werden, ohne dass es zu wesentlichen Kraft- oder Momentenstößen (Ruck) auf die führende oder positionierende Hand kommt. Alternativ zur sprunghaften Veränderung der Abtragsleistung kann es vorteilhaft sein, die Abtragsleistung relativ zum Abstand zur Arbeitsraumgrenze in mehreren Schritten oder kontinuierlich einzustellen, um eine hohe Oberflächengüte an den Grenzflächen zu erreichen.

Eine weitere beispielhafte Anwendung der werkzeugpositionsbasierten Steuerung zusammen mit einer richtungsgesteuerten Abtragsleistung ist die Folgende: Beim Bohren eines Loches mit einer elektrischen Bohrmaschine in eine Zimmerwand kann über eine kontinuierliche Lagemessung (Position/Orientierung) erkannt werden, dass sich der Bohrer knapp neben der geplanten Bohrposition befindet. In diesem Fall wird die Abtragsleistung richtungsabhängig reduziert, so dass nur noch in der Richtung zur geplanten Bohrposition Material abgetragen werden kann. Der Bohrer rutscht automatisch an die geplante Bohrposition.

Bereits aus US 7346417 ist umfangreich bekannt, welche Vorteile die Leistungssteuerung von freihandgeführten Werkzeugmaschinen auf der Basis von Positions-, Orientierungs- und Abstandsdaten besitzt. Es wird daher auf eine Wiederholung der Anwendungsfälle verzichtet. Ebenfalls ist die Kopplung der Leistungssteuerung mit direkten Messdaten eines Nervmonitorings, d. h. die Reduzierung der Abtragsleistung auf der Basis direkt sensorisch gemessener Effekte am Objekt (hier Patientenkörper), die auf der Bearbeitung des Objekts beruhen, bekannt.

Die Verstellung der Abtragsleistung, begrenzt auf eine reduzierte Zahl der Schneiden 6, wird vorteilhaft dann eingesetzt, wenn in engen Arbeitskanälen nur noch in einer Richtung Material 5 abgetragen werden soll, beispielsweise zur Herstellung von Nuten. Hier trennt das Werkzeug 1 dann nur in der voreingestellten Richtung.

Nicht immer ist es für den Werker bzw. Chirurgen ersichtlich, an welchen Stellen und in welcher Richtung noch Material 5 abgetragen werden muss. Hier ist es vorteilhaft, wenn die Wirksamkeit der Abtragsleistung richtungsabhängig visuell erkennbar ist. Dies kann beispielsweise durch eine deutlich erkennbare Einfärbung der Schneidflächen der Schneiden 6 erreicht werden. Der Anwender sieht dann selbst bei einem schnell rotierenden Effektor 4 des Werkzeugs 1 die Einfärbung immer in den Richtungen in denen eine Abtragsleistung erzielt werden kann. Es kann sinnvoll sein, einen ähnlichen Mechanismus am Schaft des Werkzeugs 1 anzubringen und hier anstatt von Schneiden 6, stumpfe oder abgerundete Flächen sichtbar werden zu lassen, die vorzugsweise vom gleichen Mechanismus freigegeben werden, um die Arbeitsrichtung deutlich zu machen.

Ebenfalls bekannt ist, dass die Berechnung der Positionsinformationen nicht nur auf der Auswertung von Markierungen (US 5389101) erfolgen kann, sondern auch direkt auf der Auswertung von Objektgeometrien (US 7079885).

Nicht nur bei pneumatischen Antrieben kann es vorteilhaft sein, die Energie zur Verstellung der Abtragsleistung und zur Steuerung der Abtragsleistung direkt aus der Antriebsenergie des Werkzeugs 1 elektrodynamisch zu erzeugen.

### Bezugszeichenliste

- 1: handgeführtes Werkzeug
- 2: Spannfutter zur Werkzeugaufnahme
- 3: Welle
- 4: Effektor
- 5: Abzutragendes Material oder Gewebe
- 6: Materialtrennende Schneiden
- 7: abtragsbegrenzte Kavität
- 8: vor Abtrag zu schützende sensible Struktur
- 9: Schneiden-Ausfahröffnung
- 10: Schneiden-Einzugsmechanismus
- 11: Schneiden-Ausfahrmechanismus
- 12: Drehgelenk
- 13: Anschlag
- 14: Schneidenschutz
- 14a: Schneidenabdeckbereich
- 15: Schneidensteueraufsatz
- 16: Stellantrieb
- 17: Lager zur Werkzeugbewegung
- 18: Lager zur Schneidenverstellung
- 19: Steuersignalleitung
- 20: Maschinenantrieb
- 21: Leistungsverstärker/Motorsteuerung
- 22: Steuerrechner
- 23: Signalleitung
- 24: Signalfunkstrecke
- 25: Schneidenverstellsteuerung
- 26: Drehzahlsensor
- 27: Werkzeug-Messmarke
- 28: Objektkörper-Messmarke
- 29: Signal- oder bildgebendes System
- 30: Signal-/Bildgeber-Messmarke
- 31: Schneidenabdeckung-Gelenk
- 32: Schneidentragende Hohlwelle
- 33: Zentrierter Stator
- 34: verstellbare Betätigungslamelle

## Patentansprüche

1. Verfahren zur signal- oder positionsbasierten Steuerung der Abtragsleistung eines handpositionierten oder handgeführten materialtrennenden Werkzeugs (1),
wobei
die materialtrennenden Schneiden (6) eines Effektors (4) und/oder mindestens eine Schneidenabdeckung (14) vor den materialtrennenden Schneiden (6) durch einen Ausfahrmechanismus und einen Einfahrmechanismus in ihrer Position und Orientierung relativ zum Effektor (4) mechanisch in mindestens zwei Lagen verstellt oder ein- und ausgefahren werden, um die Abtragsleistung der Schneiden (6) zu verändern und die Drehzahl des Effektors (4) während der Verstellung oder dem Ein- oder Ausfahren nicht oder nur unwesentlich verändert wird,
wobei die positionsbasierte Steuerung der Abtragsleistung auf einer kontinuierlich gemessenen Lage des handpositionierten oder handgeführten Werkzeugs (1) und optional deren mathematischen Ableitungen sowie räumlichen Relationen zu definierten Grenzen relativ zu dem abzutragenden Material (5) oder einer Objektkörper-Messmarke (28) und die signalbasierte Steuerung auf einem Sensorsignal oder Steuersignal beruht und wobei das Werkzeug (1) nicht am menschlichen oder tierischen Körper angewendet wird.

2. Verfahren nach Anspruch 1, **dadurch gekennzeichnet, dass** sich die Position und Orientierung des Effektors (4) durch die Verstellung der Schneiden (6) oder Schneidenabdeckung (14) nicht oder nur in der Größenordnung der Schneidenlänge der Schneiden (6) verändert und/oder dass sich die räumliche Ausdehnung des Effektors (4) durch die Verstellung der Schneiden (6) oder der Schneidenabdeckung (14) nicht oder nur in der Größenordnung der Schneidenlänge (6) oder der Schneidenabdeckung (14) verändert.

3. Verfahren nach einem der voranstehenden Ansprüche, **dadurch gekennzeichnet, dass** die erforderliche Energie zur Aufrechterhaltung der Signalverbindung und Steuerung des Stellantriebes (16) direkt aus der Bewegungsenergie oder dem Antrieb der Werkzeugbewegung gewonnen wird.

4. Verfahren nach einem der voranstehenden Ansprüche, **dadurch gekennzeichnet, dass** die mit Abtragsgeschwindigkeit rotierenden Schneiden (6) und/oder Schneidenabdeckungen (14) von einem innenliegenden Stator (33) durch mindestens eine verstellbare Betätigungslamelle (34) nach außen gedrückt werden, um so nur einen Teil der Schneiden (6) zum Abtrag freizugeben und auf diese Weise die Abtragsrichtung relativ zur Lage des Stators(33) oder der Werkzeugmessmarke (27) einzuschränken, wobei die Lage des Stators (33) relativ zu der Werkzeugmessmarke (27) bekannt ist und die Abtragsleistung des Effektors (4) durch eine Verstellung richtungsgesteuert wird.

5. Verfahren nach einem der voranstehenden Ansprüche, **dadurch gekennzeichnet, dass** die am Effektor (4) eingestellte Abtragsrichtung für den Benutzer am Werkzeug (1) visuell erkennbar ist oder akustisch oder grafisch signalisiert wird.

6. Verfahren nach einem der voranstehenden Ansprüche, **dadurch gekennzeichnet, dass** die für die zu erfolgende Materialbearbeitung optimale Abtragsrichtung durch eine Rechenvorschrift berechnet und automatisch eingestellt wird.

7. Verfahren nach einem der voranstehenden Ansprüche, **dadurch gekennzeichnet, dass** über einen Drehzahlsensor (26) die Störung eines elektromagnetischen Koordinatenmesssystems berechnet und in die Positionsmessung einbezogen wird.

8. System zur signal- oder positionsbasierten Steuerung der Abtragsleistung eines handpositionierten oder handgeführten materialtrennenden Werkzeugs (1), das einen Effektor (4) umfasst, an dem materialtrennende Schneiden (6) angeordnet sind, wobei die Schneiden (6) und/oder mindestens eine Schneidenabdeckung (14) vor den Schneiden (6) durch einen Ausfahrmechanismus und einen Einfahrmechanismus in ihrer Position und Orientierung relativ zum Effektor (4) mechanisch in mindestens zwei Lagen verstellbar ausgebildet sind, um die Abtragsleistung der Schneiden (6) zu verändern, einen Stellantrieb für die Verstellung der Schneiden (6) und/oder der mindestens einen Schneidenabdeckung (14) und einen Steuerrechner (22) zur Ansteuerung des Stellantriebs (16).

9. System nach Anspruch 8, **dadurch gekennzeichnet, dass** ein Schneidensteueraufsatz (15) und/oder ein Drehzahlsensor (26) und/oder Messmarken (27, 28, 30) angeordnet ist/sind.

10. System nach Anspruch 8, **dadurch gekennzeichnet, dass** der Schneidenausfahrmechanismus (11) durch eine direkt mit den Schneiden (6) verbundene, zweiteilig ausgebildete Keilkonstruktion oder eine indirekt Gelenkkinematik mit Drehgelenken (12) durch Verdrehung betätigbar ist.

11. System nach Anspruch 8, **dadurch gekennzeichnet, dass** der Schneideneinfahrmechanismus (10) durch schneidenverbindende Federelemente betätigbar ist.

12. System nach Anspruch 8, **dadurch gekennzeichnet, dass** die Schneiden (6) unverstellbar an einer Hohlwelle 32 befestigt sind und mit verstellbar ausgebildeten Schneidenabdeckungen (14) zusammenwirken.

13. System nach Anspruch 8, **dadurch gekennzeichnet, dass** die Schneiden (6) über ein Schneidenabdeckungsgelenk (31) mit der Schneidenabdeckung (14) verbunden sind und mit Betätigungslamellen (34) zur Verstellung zusammenwirken.

## Claims

1. Method for signal or position-based control of the removal rate of a hand-positioned or hand-guided material separating tool (1),
wherein
the material separating cutting edges (6) of an effector (4) and/or at least one cutting edge cover (14) in front of the material separating cutting edges (6) are mechanically adjusted in their position and orientation relative to the effector (4) in at least two positions or retracted and deployed by a deployment mechanism and a retraction mechanism in order to change the removal rate of the cutting edges (6), and the rotation speed of the effector (4) is not changed, or is only insignificantly changed, during the adjusting or during the retracting or the deploying,
wherein the position-based control of the removal rate is based on a continually measured position of the hand-positioned or hand-guided tool (1) and optionally on its mathematical derivatives and spatial relations to defined boundaries relative to the material (5) to be removed or to an object body measuring mark (28), and the signal-based control is based on a sensor signal or a control signal, and wherein the tool (1) is not applied on a human or animal body.

2. Method according to claim 1, **characterized in that** the position and the orientation of the effector (4) is not changed, or is only changed in the order of magnitude of the cutting length of the cutting edges (6), by adjusting the cutting edges (6) or the cutting edge cover (14), and/or **in that** the spatial extent of the effector (4) is not changed, or is only changed in the order of the cutting edge (6) length or the cutting edge cover (14), by adjusting the cutting edges (6) or the cutting edge cover (14).

3. Method according to any of the preceding claims, **characterized in that** the energy required to maintain the signal connection and to control the actuator (16) is obtained directly from kinetic energy or from the drive of the tool movement.

4. Method according to any of the preceding claims, **characterized in that** the cutting edges (6) and/or cutting edge covers (14) rotating with the removal speed are pressed outwardly by an internal stator (33) by means of at least one adjustable actuating lamella (34) so as to release only a part of the cutting edges (6) for removal and to restrict in this way the removal direction relative to the position of the stator (33) or of the tool measuring mark (27), wherein the position of the stator (33) relative to the tool measuring mark (27) is known, and wherein the removal performance of the effector (4) is directionally controlled by an adjustment.

5. Method according to any of the preceding claims, **characterized in that** the removal direction that is set at the effector is visually recognizable or acoustically or graphically signaled for the user at the tool (1).

6. Method according to any of the preceding claims, **characterized in that** the removal direction that is optimal for the material processing to be performed is calculated by a calculation rule and automatically set.

7. Method according to any of the preceding claims, **characterized in that** the disturbance of an electromagnetic coordinate measuring system is calculated via a rotation speed sensor (26) and included in the position measurement.

8. System for signal or position-based control of the removal rate of a hand-positioned or hand-guided material separating tool (1), comprising an effector (4) on which material separating cutting edges (6) are disposed, wherein the cutting edges (6) and/or at least one cutting edge cover (14) in front of the cutting edges (6) are configured to be mechanically adjustable in their position and orientation relative to the effector (4) in at least two positions by a deployment mechanism and a retraction mechanism in order to change the removal rate of the cutting edges (6), an actuator for adjusting the cutting edges (6) and/or the at least one cutting edge cover (14), and a control computer (22) for driving the actuator (16).

9. System according to claim 8, **characterized in that** a cutting edge control attachment (15) and/or a rotation speed sensor (26) and/or measuring marks (27, 28, 30) is/are disposed.

10. System according to claim 8, **characterized in that** the cutting edge deployment mechanism (11) can be actuated by means of a wedge structure designed in two pieces and directly connected to the cutting edges (6) or can be actuated by twisting by means of an indirect articulated kinematics with swivel joints (12)

11. System according to claim 8, **characterized in that** the cutting edge retraction mechanism (10) can be actuated by edge-connecting spring elements.

12. System according to claim 8, **characterized in that** the cutting edges (6) are non-adjustably fixed to a hollow shaft 32 and cooperate with cutting edge covers (14) that are configured to be adjustable.

13. System according to claim 8, **characterized in that** the cutting edges (6) are connected with the cutting edge cover (14) via a cutting edge cover joint (31) and cooperate with actuating lamellas (34) for adjustment.

## Revendications

1. Procédé de commande, sur la base d'un signal ou de la position, de la performance d'enlèvement d'un outil (1) de séparation de matière guidé ou positionné à la main,
les arêtes de coupe (6) de séparation de matière d'un effecteur (4) et/ou au moins un cache d'arête de coupe (14) se trouvant devant les arêtes de coupe (6) de séparation de matière étant réglé(e)s de façon mécanique dans leur position et leur orientation par rapport à l'effecteur (4) dans aux moins deux positions ou rétracté(e)s et sorti(e)s, par un mécanisme de sortie et de rétraction, afin de modifier la performance d'enlèvement des arêtes de coupe (6), et la vitesse de rotation de l'effecteur (4) n'étant pas modifiée ou n'étant modifiée que de façon insignifiante lors du réglage ou lors de la rétraction ou de la sortie,
la commande, sur la base de la position, de la performance d'enlèvement se basant sur une position mesurée en continu de l'outil (1) guidé ou positionné à la main et, facultativement, sur ses déduction mathématiques ainsi que sur des relations spatiales avec des limites définies par rapport à la matière (5) à enlever ou à un repère de mesure de corps d'objet (28), et la commande sur base d'un signal se basant sur un signal de capteur ou un signal de commande, et l'outil (1) n'étant pas appliqué sur un corps humain ou animal.

2. Procédé selon la revendication 1, **caractérisé en ce que** la position et l'orientation de l'effecteur (4) ne sont pas modifiées, ou ne sont modifiées que dans l'ordre de la longueur d'arête de coupe des arêtes de coupe (6) par le réglage des arêtes de coupe (6) ou du cache d'arête de coupe (14), et/ou **en ce que** l'étendue spatiale de l'effecteur (4) n'est pas modifiée, ou n'est modifiée que dans l'ordre de la longueur d'arête de coupe (6) ou du cache d'arête de coupe (14) par le réglage des arêtes de coupe (6) ou du cache d'arête de coupe (14).

3. Procédé selon l'une des revendications précédentes, **caractérisé en ce que** l'énergie nécessaire à maintenir la connexion de signal et la commande de l'actionneur (16) est obtenue directement à partir de l'énergie cinétique ou de l'entrainement du mouvement de l'outil.

4. Procédé selon l'une des revendications précédentes, **caractérisé en ce que** les arêtes de coupe (6) et/ou caches d'arête de coupe (14) tournant à la vitesse d'enlèvement sont poussé(e)s vers l'extérieur par un stator (33) intérieur au moyen d'au moins une lamelle d'actionnement (34) réglable de manière à ne débloquer qu'une partie des arêtes de coupe (6) pour l'enlèvement et à limiter ainsi la direction de l'enlèvement par rapport à la position du stator (33) ou au repère de mesure d'outil (27), la position du stator (33) par rapport au repère de mesure d'outil (27) étant connue et la performance de l'enlèvement de l'effecteur (4) étant commandée par la direction au moyen d'un réglage.

5. Procédé selon l'une des revendications précédentes, **caractérisé en ce que** la direction de l'enlèvement optimale réglée sur l'effecteur (4) est visuellement reconnaissable ou signalé de façon acoustique ou graphique sur l'outil (1) pour l'utilisateur.

6. Procédé selon l'une des revendications précédentes, **caractérisé en ce que** la direction de l'enlèvement optimale pour le traitement de la matière à effectuer est calculée par une règle de calcul et réglé automatiquement.

7. Procédé selon l'une des revendications précédentes, **caractérisé en ce que** la perturbation d'un système de mesure de coordonnées électromagnétique est calculée au moyen d'un capteur de vitesse de rotation (26) et inclus dans la mesure de position.

8. Système de commande, sur la base d'un signal ou de la position, de la performance d'enlèvement d'un outil (1) de séparation de matière guidé ou positionné à la main comprenant un effecteur (4) sur lequel sont disposées des arêtes de coupe (6) de séparation de matière, les arêtes de coupe (6) et/ou au moins un cache d'arête de coupe (14) se trouvant devant les arêtes de coupe (6) étant réalisé(e)s de façon mécanique de manière à pouvoir réglé(e)s dans leur position et leur orientation par rapport à l'effecteur (4) dans aux moins deux positions, par un mécanisme de sortie et de rétraction, afin de modifier la performance d'enlèvement des arêtes de coupe (6), un actionneur destiné à régler les arêtes de coupe (6) et/ou l'au moins un cache d'arête de coupe (14), et un ordinateur de commande (22) destiné à commander l'actionneur (16).

9. Système selon la revendication 8, **caractérisé en ce qu'**un support de commande d'arête de coupe (15) et/ou un capteur de vitesse de coupe (26) et/ou des repères de mesure (27, 28, 30) est disposé/sont disposés.

10. Système selon la revendication 8, **caractérisé en ce que** le mécanisme de sortie des arêtes de coupe (11) peut être actionné au moyen d'une structure de coin réalisée en deux pièces et reliée directement aux arêtes de coupe (6) ou peut être actionné par torsion au moyen d'une cinématique articulaire indirecte ayant des articulations tournantes (12).

11. Système selon la revendication 8, **caractérisé en ce que** le mécanisme de rétraction des arêtes de coupe (10) peut être actionné au moyen d'éléments de ressort reliant les arêtes de coupe.

12. Système selon la revendication 8, **caractérisé en ce que** les arêtes de coupe (6) sont fixées de manière non-réglable sur un arbre creux 32 et coopèrent avec des caches d'arête de coupe (14) réalisés de manière réglable.

13. Système selon la revendication 8, **caractérisé en ce que** les arêtes de coupe (6) sont reliées au cache d'arête de coupe (14) via une articulation de cache d'arête de coupe (31) et coopèrent avec des lamelles d'actionnement (34) pour le réglage.
